# EUROPÄISCHE PATENTANMELDUNG

(11) **EP 1 346 719 A1**
(43) Veröffentlichungstag der Anmeldung: **24.09.2003**
(21) Anmeldenummer: 02025423.1
(22) Anmeldetag: 15.11.2002
(51) Int. Cl.: A61K 7/13, C09B 23/00

(54) **Quinolinium-Salze enthaltende Färbemittel**

(30) Priorität: 15.03.2002 DE 10211413
(71) Anmelder: Wella Aktiengesellschaft, 64274 Darmstadt (DE)
(72) Erfinder: Sauter, Guido, Dr., 3174 Thörishaus (CH); Braun, Hans-Jürgen, Dr., 3182 Uebersdorf (CH); Duc-Reichlin, Nadia, 1569 Forel (CH)

(57) **Zusammenfassung**

Die Erfindung betrifft ein Mittel zur Färbung von Fasern (A), welches durch Vermischen zweier Komponenten -gegebenenfalls unter Zusatz eines Alkalisierungsmittels oder einer Säure- erhalten wird, und dadurch gekennzeichnet ist, dass die eine Komponente (Komponente A2) mindestens eine Verbindung mit nukleophilem Reaktionszentrum enthält und die andere Komponente (Komponente A1) mindestens ein 1-Alkylchinolinium-Derivat der Formel (Ia) oder (Ib) enthält; 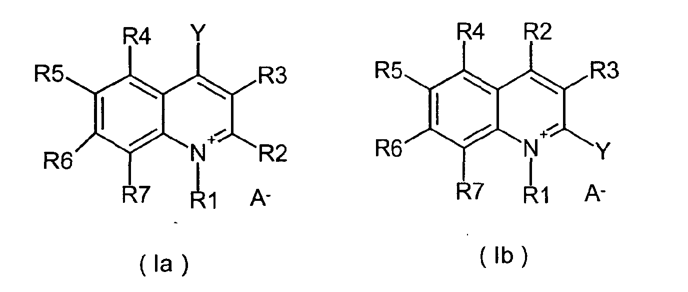 sowie einen Mehrkomponenten-Kit und ein Verfahren Färbung von Fasern.

## Beschreibung

Gegenstand der vorliegenden Erfindung sind Färbemittel, insbesondere für Keratinfasern wie zum Beispiel menschliche Haare, welche 1-Alkyl-chinolinium-Derivate mit Abgangsgruppen in Position 2 oder Position 4 enthalten.

Für das Färben von Fasern, zum Beispiel Haaren, Wolle oder Pelzen, kommen in der Regel entweder direktziehende Farbstoffe oder Oxidationsfarbstoffe, bei denen die Färbung durch Reaktion bestimmter Entwicklersubstanzen mit bestimmten Kupplersubstanzen in Gegenwart eines geeigneten Oxidationsmittels entsteht, zur Anwendung. Mit Oxidationsfarbstoffen lassen sich zwar intensive Färbungen mit guten Echtheitseigenschaften erzielen, die Entwicklung der Farbe geschieht jedoch unter dem Einfluß von Oxidationsmitteln, was in einigen Fällen Schädigungen der Faser zur Folge haben kann. Desweiteren können einige Oxidationsfarbstoffvorprodukte in Ausnahmefällen bei entsprechend disponierten Personen Allergien auslösen. Direktziehende Farbstoffe werden unter schonenderen Bedingungen appliziert, ihr Nachteil liegt jedoch darin, daß die Färbungen häufig nur über unzureichende Echtheitseigenschaften verfügen.

Aus der DE-OS 43 35 623 ist es bekannt, zur Färbung von Keratinfasern eine Kombination aus Indolinonderivaten und Verbindungen mit primären oder sekundären Aminogruppen, Heterozyklen oder aromatischen Hydroxyverbindungen einzusetzen. In der DE-OS 197 45 292 wird die Verwendung einer Kombination von bestimmten Malonaldehydderivaten, wie zum Beispiel Malonaldehyd-bis-dialkylacetalen, und Aminen oder CH-aktiven Verbindungen (beispielsweise 1-Ethyl-2-methyl-chinoliniumjodid oder 1,2-Dimethyl-chinoliniumjodid) zur Färbung von Haaren ohne Zusatz von Oxidationsmitteln beschrieben. Aus der WO 00/38633 ist die Verwendung einer Kombination von Chinolinium-Aldehyden und Aminen, Aminosäuren oder Oligopeptiden oder CH-aktiven Verbindungen zur Färbung von Haaren ohne Zusatz von Oxidationsmitteln bekannt. Weiterhin ist aus der DE-OS 199 50 404 die Verwendung einer Kombination von 1-Alkyl-methylchinolinium Salzen, Carbonylverbindungen und Alkanolamin zur Färbung von Haaren ohne Zusatz von Oxidationsmittel bekannt.

Die aus dem Stand der Technik bekannten Mittel und Verfahren zur Färbung von Fasern sind jedoch nicht in jeder Hinsicht befriedigend. Es bestand daher weiterhin ein großer Bedarf für Färbemittel, die unter milden Bedingungen, das heisst bei maximal 50°C, intensive Färbungen der Fasern mit guten Echtheitseigenschaften (Lichtechtheit, Waschechtheit, Reibechteit) ermöglichen.

Aufgabe der vorliegenden Erfindung ist es daher, ein Färbesystem zur Verfügung zu stellen, das auch ohne Zusatz von Oxidationsmitteln (wie zum Beispiel Wasserstoffperoxid) unter milden Bedingungen eine schonende und gleichzeitig intensive Färbung mit guten Echtheitseigenschaften (Lichtechtheit, Waschechtheit, Reibechtheit) ermöglicht.

Überraschenderweise wurde nunmehr gefunden, dass bei Verwendung einer Kombination (a) eines in Position 2 oder Position 4 mit einer Abgangsgruppe substituierten 1-Alkyl-chinolinium-Derivates der Formel (la) oder Formel (Ib), mit (b) einer Verbindung mit nukleophilem Reaktionszentrum, auf schonende Weise innerhalb kurzer Zeit dauerhafte Färbungen mit hervorragender Brillianz und Farbtiefe erzielt werden.

Gegenstand der vorliegenden Erfindung ist daher ein Mittel zur Färbung von Fasern, wie zum Beispiel Baumwolle Jute, Sisal, Leinen oder modifizierten Naturfasern, wie zum Beispiel Regeneratcellulose, Nitrocellulose, Alkylcellulose, Hydroxyalkylcellulose oder Acetylcellulose, oder synthetischer Fasern wie zum Beispiel Polyamidfasern, Polyacrylnitrilfasern, Polyurethanfasern und Polyesterfasern, und insbesondere keratinischen Fasern wie zum Beispiel Wolle, Seide oder Haaren -insbesondere menschlichen Haaren-, welches durch Vermischen zweier Komponenten -gegebenenfalls unter Zusatz eines Alkalisierungsmittels oder einer Säure- erhalten wird, und dadurch gekennzeichnet ist, dass die eine Komponente (Komponente A2) mindestens eine Verbindung mit nukleophilem Reaktionszentrum enthält und die andere Komponente (Komponente A1) mindestens ein 1-Alkyl-chinolinium-Derivat der Formel (la) oder (Ib) enthält, 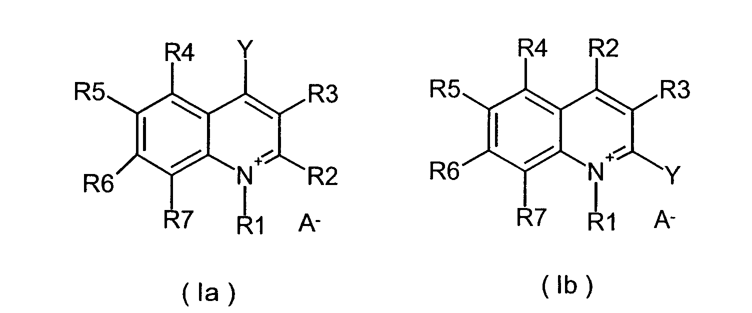 wobei gilt:
**R1** ist gleich einer geradkettigen oder verzweigten C1- bis C8-Alkylgruppe, einer C1- bis C8-Monohydroxyalkylgruppe, einer C2- bis C8-Polyhydroxyalkylgruppe oder einer C1- bis C8-Alkoxy-(C1- bis C8)-alkylgruppe;
**R2, R3, R4, R5, R6,** und **R7** können gleich oder verschieden sein und sind unabhängig voneinander gleich einem Wasserstoffatom, einer geradkettigen oder verzweigten C1- bis C4-Alkylgruppe, einer geradkettigen oder verzweigten C1- bis C4-Hydroxyalkylgruppe, einer Hydroxygruppe, einer Methoxygruppe, einer Ethoxygruppe, einer Benzylgruppe, einem Halogenatom (F, Cl, Br, J), einer Nitrogruppe, einer Nitrosogruppe, einer Cyanogruppe, einer Trifluormethylgruppe, einer -COOH -Gruppe, einer -CO₂R^{a}-Gruppe, einer -CONHR^{a}-Gruppe, einer -CON(R^{a})₂-Gruppe, einer -O(CO)R^{a}-Gruppe einer -O-SO₂CF₃-Gruppe, einer -OCH₂Aryl -Gruppe, einer -SO₂NH₂ -Gruppe, einer -SO₂CHF₂ -Gruppe, einer -SO₂CF₃ -Gruppe, einer SO₂NH₂-Gruppe, einer SO₂NHR^{a}-Gruppe, einer SO₂N(R^{a})₂-Gruppe, einer SO₂R^{a}-Gruppe, einer-NH₂-Gruppe, einer-NHR^{a}-Gruppe, einer -N(R^{a})₂ -Gruppe, einer -NHCOR^{a} -Gruppe, einer -NHCOOR^{a} -Gruppe, einer -CH₂NH₂ -Gruppe, einer -CH₂NHR^{a} -Gruppe, einer -CH₂N(R^{a})₂-Gruppe oder einer -PO(OR^{a})₂-Gruppe,
   wobei R^{a} gleich einem Wasserstoffatom, einem gegebenenfalls substituierten aromatischen Carbozyklus oder Heterozyklus oder einer C1- bis C6-Alkylgruppe ist;
**Y** steht für ein Jodatom, Bromatom, Chloratom oder eine Ethoxygruppe, Phenoxygruppe, CF₃-SO₂-O-Gruppe, Aryl-SO₂-O-Gruppe, (-SO₃)⁻Gruppe, wobei das Chloratom und die Aryl-SO₂-O-Gruppe sowie die (-SO₃)⁻ Gruppe bevorzugt sind; und
**A**⁻ ist gleich dem Anion einer organischen oder anorganischen Säure, vorzugsweise einem Chlorid-, Bromid-, Jodid-, Hydrogensulfat-, Sulfat-, Toluolsulfonat-, Benzolsulfonat-, Monomethylsulfat-, Hexafluorphosphat-, Hexafluorantimonat-, Tetrafluoroborat-, Tetraphenylborat-, Formiat-, Acetat- oder Propionation, wobei das Chloridion, dasTetrafluoroboration, das Acetation und das Hydrogensulfation besonders bevorzugt sind.

Die Verbindungen der Formeln (la) und (Ib) sind literaturbekannt oder können mit Hilfe von aus der Literatur bekannten Standardsyntheseverfahren, wie sie beispielsweise in der US-PS 3 149 105 und der GB-PS 1 102 891 oder aber von T. G. Deligeorgiev et al. in Dyes and Pigments, 1999, Seiten 49-54 und G. B. Barlin et al. in J. Chem. Soc. Perkin Trans. 2, 1975, Seiten 298-302 beschrieben werden, hergestellt werden.

Als geeignete Verbindungen der Formel (la) und Formel (Ib) können insbesondere 4-Chloro-1-ethyl-chinoliniumsalze, 4,7-Dichloro-1-ethylchinoliniumsalze, 4-Chloro-1-ethyl-7-trifluoromethyl-chinoliniumsalze, 4-Chloro-1-ethyl-6-nitrochinoliniumsalze, 4-Chloro-1-methyl-chinoliniumsalze, 4-Methoxy-1 -methyl-chinoliniumsalze, 4-Ethoxy-1-methylchinoliniumsalze, 4-Ethoxy-1-ethyl-chinoliniumsalze, 4-Jodo-1 -methyl-chinoliniumsalze, 4-Chloro-1-methyl-2-phenyl-chinoliniumsalze, 4-Chloro-1-methyl-3-[(methylphenyl-amino)sulfonyl]-chinoliniumsalze, 4-Chloro-2-[[[4-(dimethylamino)phenyl]-imino]methyl]-6-methoxy-1-methylchinoliniumsalze, 4-Chloro-1-ethyl-3-[(phenylamino)sulfonyl]-chinoliniumsalze, 4-Chloro-6-dimethylcarbamoyl-1-methyl-chinoliniumsalze, 4-Chloro-1 -ethyl-6-sulfamoyl-chinoliniumsalze, 4-Chloro-1-ethyl-7-nitro-chinoliniumsalze, 4-Chloro-1-ethyl-7-methoxy-chinoliniumsalze, 2-Chloro-1-methylchinoliniumsalze, 2,6-Dichloro-1 -methyl-chinoliniumsalze, 2-Chloro-1-methyl-4-trifluoromethanesulfonyloxy-chinoliniumsalze und 1-Ethyl-4-(toluene-4-sulfonyloxy)-chinoliniumsalze, wobei das 4-Ethoxy-1-ethylchinolinium-tetrafluoroborat, 4-Chloro-1-ethyl-chinolinium-tetrafluoroborat 4,7-Dichloro-1-ethyl-chinolinium-tetrafluoroborat, 4-Chloro-1-ethyl-7-trifluoromethyl-chinolinium-tetrafluoroborat, 4-Chloro-1-ethyl-6-nitrochinolinium-tetrafluoroborat, 4-Chloro-1-methyl-chinolinium-chlorid, 4-Jodo-1 -methyl-chinoliniumjodid, 4-Chloro-1-methyl-2-phenyl-chinoliniumtetrafluoroborat, 4-Chloro-1-methyl-3-[(methylphenyl-amino)sulfonyl]-chinolinium-methylsulfat,4-Chloro-2-[[[4-(dimethyl-amino)phenyl]imino]-methyl]-6-methoxy-1 -methyl-chinolinium-chlorid, 4-Chloro-1 -ethyl-3-[(phenylamino)-sulfonyl]-chinolinium-tetrafluoroborat, 4-Chloro-6-dimethylcarbamoyl-1 -methyl-chinolinium-tetrafluoroborat, 4-Chloro-1 -ethyl-6-sulfamoyl-chinolinium-tetrafluoroborat, 4-Chloro-1-ethyl-6-formylchinolinium-tetrafluoroborat, 4-Chloro-1-ethyl-7-nitro-chinoliniumtetrafluoroborat, 4-Chloro-1-ethyl-7-methoxy-chinolinium-tetrafluoroborat, 2-Chloro-1-methyl-chinolinium-tetrafluoroborat, 2,6-Dichloro-1-methylchinolinium-tetrafluoroborat, 1-Ethyl-4-(toluene-4-sulfonyloxy)-chinoliniumchlorid, 1-Ethyl-4-(toluene-4-sulfonyloxy)-chinolinium-tetrafluoroborat, 1-Ethyl-4-chinoliniumsulfonat und 2-Chloro-1-methyl-4-trifluoromethansulfonyloxy-chinolinium-tetrafluoroborat besonders bevorzugt sind.

Gemäss der vorliegenden Erfindung werden unter "Verbindungen mit nukleophilem Reaktionszentrum" Verbindungen verstanden, die durch Substitution der **Y**-Gruppe aus Formel (la) oder (Ib) neue Stickstoff-Kohlenstoff, Sauerstoff-Kohlenstoff oder Kohlenstoff-Kohlenstoff Bindungen bilden können. Geeignete Verbindungen mit nukleophilem Reaktionszentrum enthalten daher zum Beispiel einen Stickstoffatom oder Sauerstoffatom oder ein negativ geladenes Kohlenstoffatom (Carbanion). Beispiele für geeignete Verbindungen mit nukleophilem Reaktionszentrum gemäss der vorliegenden Erfindung sind beispielsweise aliphatische oder aromatische Verbindungen mit einer oder mehreren Aminogruppen, aliphatische oder aromatische Verbindungen mit einer Hydrazingruppe, stickstoffhaltige Heterozyklen, Aminosäuren, Oligopeptide mit 2 bis 9 Aminosäuregruppen, aromatische Hydroxyverbindungen und CH-aktive Verbindungen.

Geeignete Verbindungen mit Aminogruppen oder Hydrazingruppen sind zum Beispiel 1,4-Diamino-benzol (p-Phenylendiamin), 1,4-Diamino-2-methyl-benzol-sulfat, 1,4-Diamino-2-(2-hydroxyethyl)-benzol-sulfat, 4-Amino-2-(aminomethyl )-phenol-dihydrochlorid, 1,3-di(2,4-diaminophenoxy)-Propan-tetrahydrochlorid, 4-amino-1 -Naphthol-hydrochlorid 1,3-Diamino-4-(2-hydroxyethoxy)-benzol-sulfat, 5-Amino-2-methyl-phenol, 5-((2-hydroxyethyl)amino)-2-methoxy-Anilin-sulfat, 1,4-Diamino-2-methylbenzol-sulfat, 1,4-Diamino-2-(2-hydroxyethyl )-benzol-sulfat, 4-Amino-2-(aminomethyl)-phenol-dihydrochlorid, 1,3-Di(2,4-diaminophenoxy)-propantetrahydrochlorid, 4-Amino-1-naphthol-hydrochlorid, 1,3-Diamino-4-(2-hydroxyethoxy)-benzol-sulfat, 5-Amino-2-methyl-phenol, 5-((2-Hydroxyethyl)amino)-2-methoxy-anilin-sulfat, 1,4-Diamino-2-methyl-benzol, 1,4-Diamino-2,6-dimethyl-benzol, 1,4-Diamino-3,5-diethyl-benzol, 1,4-Diamino-2,5-dimethyl-benzol, 1,4-Diamino-2,3-dimethyl-benzol, 2-Chlor-1,4-diaminobenzol, 1,4-Diamino-2-(thiophen-2-yl)benzol, 1,4-Diamino-2-(thiophen-3-yl)benzol, 1,4-Diamino-2-(pyridin-3-yl)benzol, 2,5-Diaminobiphenyl, 1,4-Diamino-2-methoxymethyl-benzol, 1,4-Diamino-2-aminomethyl-benzol, 1,4-Diamino-2-hydroxymethyl-benzol, 1,4-Diamino-2-(2-hydroxyethoxy)-benzol, 2-(2-(Acetylamino)ethoxy)-1,4-diaminobenzol, 4-Phenylamino-anilin, 4-Dimethylamino-anilin, 4-Diethylaminoanilin, 4-Dipropylamino-anilin, 4-[Ethyl(2-hydroxyethyl)amino]-anilin, 4-[Di(2-hydroxyethyl)amino]-anilin, 4-[Di(2-hydroxyethyl)amino]-2-methylanilin, 4-[(2-Methoxyethyl)amino]-anilin, 4-[(3-Hydroxypropyl)amino]-anilin, 4-[(2,3-Dihydroxypropyl)amino]-anilin, 1,4-Diamino-2-(1 -hydroxyethyl)-benzol, 1,4-Diamino-2-(2-hydroxy-ethyl )-benzol,1,4-Diamino-2-(1-methylethyl)-benzol, 1,3-Bis[(4-amino-phenyl)(2-hydroxyethyl)amino]-2-propanol, 1,4-Bis[(4-aminophenyl)amino]-butan, 1,8-Bis(2,5-diaminophenoxy)-3,6-dioxaoctan, 4-Amino-phenol, 4-Amino-3-methyl-phenol, 4-Amino-3-(hydroxymethyl)-phenol, 4-Amino-3-fluor-phenol, 4-Methylamino-phenol, 4-Amino-2-(aminomethyl)-phenol, 4-Amino-2-(hydroxymethyl)-phenol, 4-Amino-2-fluor-phenol, 4-Amino-2-[(2-hydroxyethyl)-amino]methyl-phenol, 4-Amino-2-methyl-phenol, 4-Amino-2-(methoxymethyl)-phenol, 4-Amino-2-(2-hydroxyethyl)-phenol, 2-Amino-phenol, 2-Amino-6-methyl-phenol, 2-Amino-5-methyl-phenol, 1,2,4-Trihydroxybenzol, N-(3-Dimethylamino-phenyl)-harnstoff, 2-Amino-4-[(2-hydroxyethyl)amino]-anisol, 2,4-Diamino-1-fluor-5-methyl-benzol, 2,4-Diamino-1-methoxy-5-methyl-benzol, 2,4-Diamino-1-ethoxy-5-methyl-benzol, 2,4-Diamino-1-(2-hydroxyethoxy)-5-methyl-benzol, 2,4-Di[(2-hydroxyethyl)-amino]-1,5-dimethoxy-benzol, 1,3-Diamino-benzol, 2,4-Diamino-1-(2-hydroxyethoxy)-benzol, 1,3-Diamino-4-(2,3-dihydroxy-propoxy)-benzol, 1,3-Diamino-4-(3-hydroxypropoxy)-benzol, 1,3-Diamino-4-(2-methoxyethoxy)-benzol, 2,4-Diamino-1,5-di(2-hydroxyethoxy)-benzol, 1-(2-Aminoethoxy)-2,4-diamino-benzol, 2-Amino-1-(2-hydroxyethoxy)-4-methylaminobenzol, 2,4-Diaminophenoxy-essigsäure, 3-[Di(2-hydroxy-ethyl)amino]-anilin, 4-Amino-2-di[(2-hydroxyethyl)amino]-1-ethoxy-benzol, 5-Methyl-2-(1 -methylethyl)-phenol, 3-[(2-Hydroxyethyl)amino]-anilin, 3-[(2-Aminoethyl)amino]-anilin, 1,3-Di(2,4-diaminophenoxy)-propan, Di(2,4-diaminophenoxy)-methan,1,3-Diamino-2,4-dimethoxy-benzol, 2,6-Bis(2-hydroxyethyl)amino-toluol, 4-Hydroxyindol, 3-Dimethylamino-phenol, 3-Diethylamino-phenol, 5-Amino-2-methyl-phenol, 5-Amino-4-fluor-2-methylphenol, 5-Amino-4-methoxy-2-methyl-phenol, 5-Amino-4-ethoxy-2-methylphenol, 3-Amino-2,4-dichlor-phenol, 5-Amino-2,4-dichlor-phenol, 3-Amino-2-methyl-phenol, 3-Amino-2-chlor-6-methyl-phenol, 3-Amino-phenol, 2-[(3-Hydroxyphenyl)amino]-acetamid, 5-[(2-Hydroxy-ethyl)amino]-4-methoxy-2-methyl-phenol, 5-[(2-Hydroxyethyl)amino]-2-methyl-phenol, 3-[(2-Hydroxyethyl)amino]-phenol, 3-[(2-Methoxyethyl)-amino]-phenol, 5-Amino-2-ethyl-phenol, 5-Amino-2-methoxy-phenol, 2-(4-Amino-2-hydroxyphenoxy)-ethanol, 5-[(3-Hydroxypropyl)amino]-2-methyl-phenol, 3-[(2,3-Dihydroxypropyl)amino]-2-methyl-phenol, 3-[(2-Hydroxyethyl)-amino]-2-methyl-phenol, 5-Amino-4-chlor-2-methyl-phenol, 1-Naphthol, 2-Methyl-1-naphthol, 1,5-Dihydroxy-naphthalin, 1,7-Dihydroxy-naphthalin, 2,3-Dihydroxy-naphthalin, 2,7-Dihydroxy-naphthalin, 2-Methyl-1-naphtholacetat, 1,3-Dihydroxy-benzol, 1-Chlor-2,4-dihydroxy-benzol, 2-Chlor-1,3-dihydroxy-benzol, 1,2-Dichlor-3,5-dihydroxy-4-methyl-benzol, 1,5-Dichlor-2,4-dihydroxy-benzol, 1,3-Dihydroxy-2-methyl-benzol, 3,4-Methylendioxyphenol, 3,4-Methylendioxy-anilin, 5-[(2-Hydroxyethyl)amino]-1,3-benzodioxol, 6-Brom-1-hydroxy-3,4-methylendioxy-benzol, 3,4-Diamino-benzoesäure, 2-Aminobenzoesäure, 3-Aminobenzoesäure, 4-Aminobenzoesäure, 2,3-Diaminobenzoesäure, 2,4-Diaminobenzoesäure, 2,5-Diaminobenzoesäure, 3,4-Diamino-benzoesäure, 3,5-Diamino-benzoesäure, 4-Aminosalicylsäure, 5-Amino-salicylsäure, 3-Amino-4-hydroxy-benzoesäure, 4-Amino-3-hydroxy-benzoesäure, 2-Aminobenzolsulfonsäure, 3-Aminobenzolsulfonsäure, 4-Aminobenzolsulfonsäure, 3-Amino-4-hydroxybenzolsulfonsäure, 4-Amino-3-hydroxynaphthalin-1-sulfonsäure, 6-Amino-7-hydroxynaphthalin-2-sulfonsäure, 7-Amino-4-hydroxynaphthalin-2-sulfonsäure, 4-Amino-5-hydroxynaphthalin-2,7-disulfonsäure, 3-Amino-2-naphthoesäure, 3-Aminophthalsäure, 5-Aminoisophthalsäure, 1,3,5-Triaminobenzol, 1,2,4-Triaminobenzol, 1,2,4,5-Tetraaminobenzol, 2,4,5-Triaminophenol, Pentaaminobenzol, Hexaminobenzol, 2,4,6-Triaminoresorcin, 4,5-Diaminobrenzcatechin, 4,6-Diaminopyrogallol, 3,5- Diamino-4-hydroxybrenzcatechin, aromatische Aniline oder Phenole mit einem weiteren aromatischen Rest, wie beispielsweise 4,4'-Diaminostilben, 4,4'-Diaminostilben-2,2'-disulfonsäuremono-Na-Salz oder 4,4'-Diaminostilben-2,2'-disulfonsäuredi-Na-Salz, 4,4,-Diaminodiphenylmethan, 4,4,-Diaminodiphenylsulfid, 4,4,-Diaminodiphenylsulfoxid, 4,4,-Diaminodiphenylamin, 4,4,-Diaminodiphenylamin-2-sulfonsäure, 4,4'-Diaminobenzophenon, 4,4'-Diaminobenzophenondiphenylether, 3,3',4,4-Tetraamino-diphenyl, 3,3',4,4'-Tetraamino-benzophenon, 1,3-Bis-(2,4-diaminophenoxy)-propan, 1,8-Bis-(2,5-diaminophenoxy)-3,6-dioxaoctan, 1,3-Bis-(4-aminophenylamino)-propan, 1,3-Bis-(4-aminophenylamino)-2-propanol, 1,3-Bis-[N-(4-aminophenyl)-2-hydroxyethylamino]-2-propanol, N,N-Bis-[2-(4-aminophenoxy)-ethyl]-methylamin, Phenyl-hydrazin, Phenyl-hydrazin-hydrochlorid, 2,4-Dinitro-1-hydrazinobenzol, 4-Nitrophenylhydrazin, 2-Nitrophenylhydrazin, 4-Methoxyphenylhydrazin, 4-Methoxy-phenylhydrazin-hydrochlorid, p-Tolylhydrazin, p-Tolylhydrazin-hydrochlorid, 4-Fluor-phenylhydrazin, 4-Fluorphenylhydrazin-hydrochlorid, 4-Isopropylphenylhydrazin, 4-Isopropylphenylhydrazin-hydrochlorid, 4-Brom-phenylhydrazin, 4-Brom-phenylhydrazin-hydrochlorid, o-Tolylhydrazin-hydrochlorid, 2,3-Dimethylphenylhydrazin-hydrochlorid-hydrat, 2,4-Dimethyl-phenylhydrazin-hydrochlorid, 2,5-Dimethylphenylhydrazin-hydrochlorid, o-Tolylhydrazin, 2,3-Dimethylphenylhydrazin, 2,4-Dimethylphenylhydrazin, 2,5-Dimethylphenylhydrazin, 2,4,6-Trichlor-phenylhydrazin, N-Methyl-N-phenylhydrazin, N,N-Diphenylhydrazin-hydrochlorid und N-Phenyl-1,4-phenyendiamin.

Geeignete stickstoffhaltige heterozyklische Verbindungen sind zum Beispiel 2-Aminopyridin, 3-Aminopyridin, 4-Aminopyridin, 2-Amino-3-hydroxy-pyridin, 2,6-Diaminopyridin, 2,5-Diaminopyridin, 2,3-Diaminopyridin, 2-Dimethylamino-5-aminopyridin, 2-Methylamino-3-amino-6-methoxy-pyridin, 2,3-Diamino-6-methoxypyridin, 2,6-Dimethoxy-3,5-diaminopyridin, 2,4,5-Triaminopyridin, 2,6-Dihydroxy-3,4-dimethylpyridin, 3-Amino-6-methoxy-2-(methylamino)-pyridin, 2,6-Diamino-3,5-dimethoxypyridin, 3,5-Diamino-2,6-dimethoxy-pyridin, 2,4-Dihydroxy-5,6-diaminopyrimidin, 4,5,6-Triamino-pyrimidin, 4-Hydroxy-2,5,6-triamino-pyrimidin, 2-Hydroxy-4,5,6-triamino-pyrimidin, 2,4,5,6-Tetraamino-pyrimidin, 2,5,6-Triamino-4-(1H)-pyrimidon, 2-Methyl-amino-4,5,6-triamino-pyrimidin, 2,4-Diamino-pyrimidin, 4,5-Diamino-pyrimidin, 2-Amino-4-methoxy-6-methyl-pyrimidin, 2,6,8-Trihydroxy-purin, 4,5-Diamino-1-(2-hydroxyethyl)-1 H-pyrazol-sulfat, 4,5-Diamino-1-(2-hydroxyethyl)-1 H-pyrazol, 4,5-Diamino-1-(1-methylethyl)-1 H-pyrazol, 4,5-Diamino-1-[(4-methylphenyl)methyl]-1 H-pyrazol, 1-[(4-Chlorphenyl)methyl]-4,5-d iamino- 1H-pyrazol, 4,5-Diamino-1-methyl-1 H-pyrazol, 3,5-Diaminopyrazol, 3,5-Diamino-1,2,4-triazol, 3-Aminopyrazol, 3-Amino-5-hydroxypyrazol, 2-Aminochinolin, 3,4-Dihydro-6-hydroxy-1,4(2H)-benzoxazin, 6-Amino-3,4-dihydro-1,4(2H)-benzoxazin, 3-Methyl-1-phenyl-5-pyrazolon, 5,6-Dihydroxy-indol, 5,6-Dihydroxy-indolin, 5-Hydroxy-indol, 6-Hydroxy-indol, 7-Hydroxy-indol, 2,3-Indolindion. 3-Aminochinolin, 8-Aminochinolin, 4-Amino-methylchinolin, 2-Aminonicotinsäure, 6-Aminonicotinsäure, 5-Amino-isochinolin, 5-Aminoindazol, 6-Aminoindazol, 5-Aminobenzimidazol, 7-Aminobenzimidazol, 7-Amino- benzothiazol, 5-Amino-benzothiazol, 2,5-Dihydroxy-4-morpholinoanilin sowie Indol- und Indolinderivaten, wie 4-Aminoindol, 5-Aminoindol, 6-Aminoindol, 7-Aminoindol, 5,6-Dihydroxy-indol, 5,6-Dihydroxyindolin und 4-Hydroxyindolin.

Die vorgenannten Verbindungen können sowohl in freier Form als auch in Form ihrer physiologisch verträglichen Salze, beispielsweise als Salze anorganischer Säuren, wie Salzsäure oder Schwefelsäure, eingesetzt werden.

Als Aminosäuren kommen sowohl natürlich vorkommende als auch synthetische Aminosäuren in Frage, insbesondere Arginin, Histidin, Tyrosin, Phenylalanin, Dihydroxyphenylalanin, Ornithin, Lysin und Tryptophan.

Als Oligopeptide können alle natürlich vorkommenden oder synthetischen Oligopeptide, sowie die-in Polypepid- oder-Proteinhydrolysaten enthaltenen Oligopeptide eingesetzt werden, sofern sie über eine für die Anwendung in den erfindungsgemäßen Färbemitteln ausreichende Wasserlöslichkeit verfügen. Als Beispiele können folgende Verbindungen genannt werden: Glutathion oder die in den Hydrolysaten von Kollagen, Keratin, Casein, Elastin, Sojaprotein, Weizengluten oder Mandelprotein enthaltenen Oligopeptide. Bevorzugt ist hierbei die gemeinsame Verwendung der Oligopeptide mit Verbindungen mit primärer oder sekundärer Aminogruppe oder mit aromatischen Hydroxyverbindungen.

Geeignete aromatische Hydroxyverbindungen sind zum Beispiel 2-Methylresorcin, 4-Methylresorcin, 5-Methylresorcin, 2,5-Dimethylresorcin, Resorcin, 3-Methoxyphenol, Brenzkatechin, Hydrochinon, Pyrogallol, Phloroglucin, Hydroxyhydrochinon, 2-Methoxyphenol, 3-Methoxyphenol, 4-Methoxy-phenol, 3-Dimethylaminophenol, 2-(2-Hydroxyethyl)-phenol, 3,4-Methylendioxyphenol, 2,4-Dihydroxy-benzoesäure, 3,4-Dihydroxy-benzoesäure, 2,4-Dihydroxy-phenylessigsäure, 3,4-Dihydroxy-phenylessigsäure, Gallussäure, 2,4,6-Trihydroxybenzoesäure, 2,4,6-Trihydroxy-acetophenon, 2-Chlorresorcin, 4-Chlorresorcin, 1-Naphthol, 1,5-Dihydroxynaphthalin, 2,3-Dihydroxy-naphthalin, 2,7-Dihydroxynaphthalin, 4-hydroxy-2-naphthalinsulfonsäure und 3,6-Dihydroxy-2,7-naphthalinsulfonsäure.

Als geeignete CH-aktive Verbindungen können beispielsweise genannt werden: 1-Ethyl-2-methylchinolinium-jodid, 1 -Ethyl-2-methylchinoliniumchlorid, 1-Ethyl-2-methylchinolinium-tetrafluoroborat, 1-Ethyl-2-methylchinolinium-methylsulfat, 1 -Methyl-2-methylchinolinium-jodid, 1-Methyl-2-methylchinolinium-chlorid, 1-Methyl-2-methylchinolinium-tetrafluoroborat, 1-Methyl-2-methylchinolinium-methylsulfat, 1-Ethyl-4-methylchinoliniumjodid, 1-Ethyl-4-methylchinolinium-chlorid, 1-Ethyl-4-methylchinoliniumtetrafluoroborat, 1-Ethyl-4-methylchinolinium-methylsulfat, 1-Methyl-4-methylchinolinium-jodid, 1 -Methyl-4-methylchinolinium-chlorid, 1-Methyl-4-methylchinolinium-tetrafluoroborat, 1 -Methyl-4-methylchinoliniummethylsulfat, 1,2,3,3-Tetramethyl-3H-indolium-hydrogensulfat, 3-Ethyl-1,2,3-trimethyl-3H-indolium-perchlorat, 1,2,3,3,5-Pentamethyl-3Hindolium-jodid, 1,2,3,3,7-Pentamethyl-3H-indolium-tetrafluoroborat, 1,2,3,3,6,7-Hexamethyl-3H-indolium-tetrafluoroborat, 1,2,3,3,5,7-Hexamethyl-3H-indolium-tetrafluoroborat, 1,2,3,3,4,7-Hexamethyl-3H-indolium-tetrafluoroborat, 5-Fluoro-1,2,3,3-tetramethyl-3H-indolium-jodid, 5-lsopropyl-1,2,3,3-tetramethyl-3Hindolium-jodid, 5-Nitro-1,3,3-trimethyl-2-methylen-indolin, 5-Methoxy-1,2,3,3-tetramethyl-3H-indolium-jodid, 5-Methoxy-6-nitro-1,2,3,3-tetramethyl-3H-indolium-chlorid, 5-Hydroxy-1,2,3,3-tetramethyl-3Hindolium-jodid, 5-N-acetylamino-1,2,3,3-tetramethyl-3H-indolium-acetat, 2,3-dimethyl-benzothiazolium-jodid, 2,3-Dimethyl-benzothiazolium-ptoluolsulfonat, Rhodanin, Rhodanin-3-essigsäure, Barbitursäure, Thiobarbitursäure, 1,3-Dimethyl-thiobarbitursäure, 1,3-Diethylthiobarbitursäure, Oxindol, 3-Indoxylacetat.

Die Verbindungen der Formel (la) oder (Ib) werden bis kurz vor der Anwendung von den Verbindungen mit nukleophilem Reaktionszentrum getrennt aufbewahrt. Das erfindungsgemäße Färbemittel besteht daher in der Regel aus zwei Komponenten, nämlich einer Farbträgermasse (A1), welche die Verbindungen der Formel (la) oder (Ib) und gegebenenfalls direktziehende Farbstoffe enthält, und einer weiteren Farbträgermasse (A2), welche die Verbindungen mit nukleophilem Reaktionszentrum und gegebenenfalls direktziehende Farbstoffe enthält. Diese beiden Komponenten werden unmittelbar vor der Anwendung zu einem gebrauchsfertigen Färbemittel (A) vermischt und sodann auf die zu färbende Faser aufgetragen. Selbstverständlich ist es auch möglich, dass eine oder beide Komponenten (A1) und (A2) aus mehreren Einzelkomponenten bestehen, welche vor der Anwendung miteinander vermischt werden. Besonders bevorzugt ist jedoch ein 2-Komponenten-Kit, bestehend aus einem Mittel der Komponente (A1) und einem Mittel der Komponente (A2).

Obwohl eine Verwendung des vorgenannten Mittels ohne den Zusatz von Oxidationsmitteln wegen der besseren Schonung der Faser bevorzugt ist, ist eine Verwendung des vorgenannten Färbemittels in Verbindung mit Oxidationsmitteln prinzipiell möglich, beispielsweise wenn eine gleichzeitige Bleichung der Faser gewünscht wird oder wenn dem Färbemittel übliche Oxidationsfarbstoffvorstufen zugesetzt werden sollen.

Die Verbindungen der Formel (la) oder (Ib) und die Verbindung mit nukleophilem Reaktionszentrum sind in der jeweiligen Farbträgermasse (Komponente (A1) beziehungsweise Komponente (A2)) jeweils in einer Gesamtmenge von etwa 0,02 bis 20 Gewichtsprozent, vorzugsweise 0,2 bis 10 Gewichtsprozent, enthalten, wobei in dem durch Vermischen der Komponenten (A1) und (A2) erhaltenen gebrauchsfertigen Färbemittel (A) die Verbindungen der Formel (la) oder (Ib) und die Verbindung mit nukleophilem Reaktionszentrum jeweils in einer Gesamtmenge von etwa 0,01 bis 10 Gewichtsprozent, vorzugsweise 0,1 bis 5 Gewichtsprozent, enthalten sind.

Weiterhin kann das erfindungsgemäße Färbemittel gegebenenfalls zusätzlich übliche, physiologisch unbedenkliche, direktziehende Farbstoffe aus der Gruppe der sauren oder basischen Farbstoffe, Nitrofarbstoffe, Azofarbstoffe, Chinonfarbstoffe und Triphenylmethanfarbstoffe enthalten. Die direktziehenden Farbstoffe können in der Komponente (A1) und/oder der Komponente (A2) jeweils in einer Gesamtenge von etwa 0,02 bis 20 Gewichtsprozent, vorzugsweise 0,2 bis 10 Gewichtsprozent, eingesetzt werden, wobei die Gesamtmenge an direktziehenden Farbstoffen in dem durch Vermischen der Komponenten (A1) und (A2) erhaltenen gebrauchsfertigen Färbemittel (A) etwa 0,01 bis 10 Gewichtsprozent, vorzugsweise 0,1 bis 5 Gewichtsprozent, beträgt.

Vorzugsweise ist das erfindungsgemäße Mittel frei von Aldhyden und Ketonen.

Die Zubereitungsform für das gebrauchsfertige Färbemittel (A) sowie die Komponenten (A1) und (A2) kann beispielsweise eine Lösung, insbesondere eine wässrige oder wässrig-alkoholische Lösung sein. Weitere geeignete Zubereitungsformen sind eine Creme, ein Gel, ein Schaum oder eine Emulsion. Ihre Zusammensetzung stellt eine Mischung der Verbindungen der Formeln (la) oder (Ib) und/oder der Verbindungen mit nukleophilem Reaktionszentrum mit den für solche Zubereitungen üblichen Zusätzen dar.

Übliche in Färbemitteln verwendete Zusätze in Lösungen, Cremes, Emulsionen, Gelen oder Schäumen sind zum Beispiel Lösungsmittel wie Wasser, niedere aliphatische Alkohole, beispielsweise Ethanol, n-Propanol und Isopropanol oder Glykole wie Glycerin und 1,2-Propandiol, weiterhin Netzmittel oder Emulgatoren aus den Klassen der anionischen, kationischen, amphoteren oder nichtionogenen oberflächenaktiven Substanzen wie Fettalkoholsulfate, oxethylierte Fettalkoholsulfate, Alkylsulfonate, Alkylbenzolsulfonate, Alkyltrimethylammoniumsalze, Alkylbetaine, oxethylierte Fettalkohle, oxethylierte Nonylphenole, Fettsäurealkanolamide, oxethylierte Fettalkohole, oxethylierte Nonylphenole, Fettsäurealkanolamide, oxethylierte Fettsäureester, ferner Verdicker wie höhere Fettalkohole, Stärke oder Cellulosederivate, Parfüme, Haarvorbehandlungsmittel, Konditionierer, Haarquellmittel, Konservierungsstoffe, weiterhin Vaseline, Paraffinöl und Fettsäuren sowie außerdem Pflegestoffe wie kationische Harze, Lanolinderivate, Cholesterin, Pantothensäure und Betain. Die erwähnten Bestandteile werden in den für solche Zwecke üblichen Mengen verwendet, zum Beispiel die Netzmittel und Emulgatoren in Konzentrationen von etwa 0,5 bis 30 Gewichtsprozent (bezogen auf die Farbträgermasse), die Verdicker in einer Menge von etwa 0,1 bis 30 Gewichtsprozent (bezogen auf die Farbträgermasse) und die Pflegestoffe in einer Konzentration von etwa 0,1 bis 5 Gewichtsprozent (bezogen auf die Farbträgermasse).

Der pH-Wert des gebrauchsfertigen Färbemittels beträgt in der Regel etwa 3 bis 11, vorzugsweise etwa 5 bis 11, wobei ein pH-Wert von 7 bis 10 besonders bevorzugt ist. Der pH-Wert des gebrauchsfertigen Färbemittels (A) ergibt sich bei der Mischung der die Verbindungen der Formel (la) oder (Ib) enthaltenden Komponente (A1) mit der die Verbindung mit nukleophilem Reaktionszentrum enthaltenden Komponente (A2) in Abhängigkeit vom pH-Wert der Komponenten (A1) und (A2) sowie dem Mischungsverhältnis dieser beiden Komponenten. Falls erforderlich kann nach dem Vermischen der Komponenten (A1) und (A2) der pH-Wert des gebrauchsfertigen Färbemittels (A) durch den Zusatz eines geeigneten Alkalisierungsmittels oder einer Säure auf den gewünschten Wert eingestellt werden.

Zur Einstellung des pH-Wertes des gebrauchsfertigen Färbemittels (A) sowie der Komponente (A1) oder (A2) können alkalisierende Mittel wie zum Beispiel Alkanolamine, Alkylamine, Alkalihydroxide oder Ammoniumhydroxid, Alkalicarbonate oder Ammoniumcarbonate und Ammoniaklösungen oder Säuren wie zum Beispiel Milchsäure, Essigsäure, Weinsäure, Phosphorsäure, Salzsäure, Zitronensäure, Ascorbinsäure und Borsäure, verwendet werden.

Das gebrauchsfertige Färbemittel (A) wird unmittelbar vor der Anwendung durch Vermischen der die Verbindungen der Formel (la) oder (Ib) enthaltenden Komponente (A1) mit der die Verbindung mit nukleophilem Reaktionszentrum enthaltenden Komponente (A2) (gegebenenfalls unter Zusatz eines alkalisierenden Mittels oder einer Säure) hergestellt und sodann auf die Faser aufgetragen. Je nach gewünschter Farbtiefe läßt man diese Mischung 5 bis 60 Minuten, vorzugsweise 15 bis 30 Minuten, bei einer Temperatur von 20 bis 50 Grad Celsius, insbesondere bei 30 bis 40 Grad Celsius einwirken. Anschließend wird die Faser mit Wasser gespült und gegebenenfalls mit einem Shampoo gewaschen.

Das erfindungsgemässe Färbemittel ermöglicht eine breite Palette von Farbnuancen im Bereich von gelb-rot bis blau und braunschwarz mit hervorragenden Echtheitseigenschaften, wobei auch ohne Zusatz von Oxidationsmitteln eine schonende, gleichmässige und dauerhafte Färbung der Fasern, insbesondere von Keratinfasern, wie zum Beispiel menschlichen Haaren, erzielt wird.

Die nachfolgenden Beispiele sollen den Gegenstand näher erläutern, ohne ihn auf diese Beispiele zu beschränken.

### Beispiele

| **Beispiele 1 bis 7** Haarfärbemittel | |
|---|---|
| Komponente (A1): | |
| Verbindung der Formel (la) oder (Ib) | Menge gemäss Tabelle 1 |
| Decyl-Glucoside | 4,0 g |
| Ethylendiaminotetraacetat-Dinatriumsalz | 0,2 g |
| Ethanol | 5,0 g |
| Wasser, vollentsalzt | ad 100,0 g |

| Komponente (A2): | |
|---|---|
| Verbindung mit nukleophilem | |
| Reaktionszentrum | Menge gemäss Tabelle 1 |
| Decyl-Glucoside | 4,0 g |
| Ethylendiaminotetraacetat-Dinatriumsalz | 0,2 g |
| Ethanol | 5,0 g |
| 25%ige wässrige Ammoniaklösung | 6,0 g |
| Wasser, vollentsalzt | ad 100,0 g |

Die Komponente (A1) und die Komponente (A2) werden im Verhältnis 1:1 miteinander vermischt. Das so erhaltene gebrauchsfertige Haarfärbemittel (A) wird auf das Haar aufgetragen und mit einem Pinsel gleichmäßig verteilt. Nach einer Einwirkungszeit von 30 Minuten bei 40 °C wird das Haar mit einem Shampoo gewaschen, anschließend mit lauwarmem Wasser gespült und sodann getrocknet.

Die Einsatzmengen sowie die Färberesultate sind in Tabelle 1 zusammengefasst.

| **Beispiele 8 bis 14** Haarfärbemittel | |
|---|---|
| Komponente (A1): | |
| Verbindung der Formel (la) oder (Ib) | Menge gemäss Tabelle 2 |
| Cocoamidopropyl-betain | 7,5 g |
| Ethanol | 5,0 g |
| Wasser, vollentsalzt | ad 100,0 g |

| Komponente (A2): | |
|---|---|
| Verbindung mit nukleophilem Reaktionszentrum | Menge gemäss Tabelle 2 |
| Cocoamidopropyl-betain | 7,5 g |
| Ethanol | 5,0 g |
| 25%ige wässrige Ammoniaklösung | 6,0 g |
| Wasser, vollentsalzt | ad 100,0 g |

Die Komponente (A1) und die Komponente (A2) werden im Verhältnis 1:1 miteinander vermischt. Das so erhaltene gebrauchsfertige Haarfärbemittel (A) wird auf das Haar aufgetragen und mit einem Pinsel gleichmäßig verteilt. Nach einer Einwirkungszeit von 30 Minuten bei 40 °C wird das Haar mit einem Shampoo gewaschen, anschließend mit lauwarmem Wasser gespült und sodann getrocknet.

Die Einsatzmengen sowie die Färberesultate sind in Tabelle 2 zusammengefasst.

| **Beispiele 15 bis 24** Haarfärbemittel | |
|---|---|
| Komponente (A1): | |
| Verbindung der Formel (la) oder (Ib) | Menge gemäss Tabelle 3 |
| Cetylstearylalkohol | 12,0 g |
| Polyethylenglykoloktadecylether | |
| (Brij® 78 P der Firma Fluka) | 2,5 g |
| Wasser, vollentsalzt | ad 100,0 g |

| Komponente (A2): | |
|---|---|
| Verbindung mit nukleophilem | |
| Reaktionszentrum | Mengenangaben gemäss Tabelle 1-3 |
| Cetylstearylalkohol | 12,0 g |
| Polyethylenglykoloktadecylether | |
| (Brij® 78 P der Firma Fluka) | 2,5 g |
| Wasser, vollentsalzt | ad 100,0 g |

Zur Herstellung der Komponente (A1) bzw. (A2) wird jeweils der Cetylstearylalkohol bei 80 °C geschmolzen. Der Polyethylenglykoloktadecylether wird mit 95 % des Wassers auf 80 °C erhitzt und zum geschmolzenen Cetylstearylalkohol gegeben. Anschließend wird gerührt bis sich eine Creme ergibt. Bei Raumtemperatur wird die Verbindung mit nukleophilem Reaktionszentrum bzw. die Verbindung der Formel (Ia)/(Ib) zusammen mit dem restlichen Wasser zugegeben.

Die Komponente (A1) und die Komponente (A2) werden im Verhältnis 1:1 miteinander vermischt. Der pH-Wert der so erhaltenen Mischung wird erforderlichenfalls durch Zugabe einer 25%igen wässrigen Ammoniaklösung auf den in Tabelle 3 angegebenen pHₘ-Wert eingestellt. Das so erhaltene gebrauchsfertige Haarfärbemittel (A) wird auf das Haar aufgetragen und mit einem Pinsel gleichmäßig verteilt. Nach einer Einwirkungszeit von 30 Minuten bei 40 °C wird das Haar mit einem Shampoo gewaschen, anschließend mit lauwarmem Wasser gespült und sodann getrocknet.

Die Einsatzmengen sowie die Färberesultate sind in Tabelle 3 zusammengefasst.

Die in den vorliegenden Beispielen angegebenen L*a*b*-Farbmesswerte wurden mit einem Farbmessgerät der Firma Minolta, Typ Chromameter II, ermittelt. Hierbei steht der L-Wert für die Helligkeit (das heißt je geringer der L-Wert ist, umso größer ist die Farbintensität), während der a-Wert ein Maß für den Rotanteil ist (das heißt je größer der a-Wert ist, umso größer ist der Rotanteil). Der b-Wert ist ein Maß für den Blauanteil der Farbe, wobei der Blauanteil umso größer ist, je negativer der b-Wert ist.

Alle Prozentangaben in der vorliegenden Anmeldung stellen, sofern nicht anders angegeben, Gewichtsprozente dar.

**Tabelle 1:**

| Haarfärbemittel | | | | | |
|---|---|---|---|---|---|
| **Bsp. Nr.** | **Komponente (A1) /** | **Farbton** | **Farbmeßwerte** | | |
| | **Komponente (A2)** | | **L** | **a** | **b** |
| **1** | (A1) 4-Chlor-1 -ethyl-chinolinium-tetrafluoroborat: 0,70 g (A2) 1,4-Diamino-benzol: 0,27 g | gelb | 77,00 | -1,12 | +67,37 |
| **2** | A1) 4-Chlor-1-ethyl-chinolinium-tetrafluoroborat: 0,70 g A2) 1,4-Diamino-2-methyl-benzol-sulfat: 0,55 g | gelb | 67,61 | +0,28 | +54,88 |
| **3** | (A1) 4-Chlor-1 -ethyl-chinolinium-tetrafluoroborat: 0,70 g (A2) 1,4-Diamino-2-(2-hydroxy-ethyl)-benzol-sulfat: 0,63 g | gelb | 71,23 | +0,02 | +61,12 |
| **4** | (A1) 4-Chlor-1-ethyl-chinolinium-tetrafluoroborat: 0,70 g (A2) 4,5-Diamino-1-(2-hydroxy-ethyl)-1 H-pyrazol-sulfat: 0,60 g | gelb | 76,13 | +2,24 | +34,69 |
| **5** | (A1) 4-Chlor-1 -ethyl-chinolinium-tetrafluoroborat: 0,70 g (A2) 4-Amino-2-(aminomethyl)-phenol-dihydrochlorid: 0,53 g | gelb | 60,71 | +3,10 | +26,46 |
| **6** | (A1) 4-Chlor-1-ethyl-chinolinium-tetrafluoroborat: 0,70 g (A2) 1,3-di(2,4-diaminophenoxy)-Propan-tetrahydrochlorid: 1,09 g | gelb | 69,04 | -1,32 | +40,76 |
| **7** | (A1) 4-Chlor-1-ethyl-chinolinium-tetrafluoroborat: 0,70 g (A2) 4-amino-1-Naphthol-hydrochlorid: 0,49 g | braun | 52,01 | +7,87 | +21,33 |

**Tabelle 2:**

| Haarfärbemittel | | | | | |
|---|---|---|---|---|---|
| Bsp. Nr. | **omponente (A1)** | **Farbton** | **Farbmeßwerte** | | |
| | **omponente (A2)** | | **L** | **a** | **b** |
| **8** | A1) 4-Chlor-1-ethyl-chinolinium-etrafluoroborat: 0,70 g A2) 1,4-Diamino-benzol: 0,27 g | gelb | 73,05 | -0,97 | +61,18 |
| **9** | A1) 4-Chlor-1-ethyl-chinolinium-etrafluoroborat: 0,70 g A2) 1,4-Diamino-2-methyl-benzol-ulfat: 0,55 g | gelb | 70,54 | +1,86 | +61,19 |
| **10** | A1) 4-Chlor-1-ethyl-chinolinium-etrafluoroborat: 0,70 g A2) 1,4-Diamino-2-(2-hydroxy-thyl)-benzol-sulfat: 0,63 g | gelb | 72,97 | -0,45 | +61,60 |
| **11** | 1) 4-Chlor-1-ethyl-chinolinium-etrafluoroborat: 0,70 g 2) 4,5-Diamino-1-(2-hydroxy-thyl)-1 H-pyrazol-sulfat: 0,60 g | gelb | 73,11 | +6,03 | +28,58 |
| **12** | (A1) 4-Chlor-1-ethyl-chinolinium-tetrafluoroborat: 0,70 g (A2) 4-Amino-2-(aminomethyl)-phenol-dihydrochlorid: 0,53 g | gelb | 63,02 | +2,81 | 27,44 |
| **13** | (A1) 4-Chlor-1-ethyl-chinolinium-tetrafluoroborat: 0,70 g (A2) 1,3-di(2,4-diaminophenoxy)-Propan-tetrahydrochlorid: 1,09 g | gelb | 68,16 | -1,10 | 32,82 |
| **14** | (A1) 4-Chlor-1-ethyl-chinolinium-tetrafluoroborat: 0,70 g (A2) 4-amino-1-Naphthol-hydrochlorid: 0,49 g | braun | 56,60 | +8,49 | 24,73 |

**Tabelle 3:**

| Haarfärbemittel | | | | | |
|---|---|---|---|---|---|
| **Bsp.Nr.** | **Komponente (A1)/** | **Farbton** | **Farbmeßwerte** | | |
| | **Komponente (A2)** | | | | |
| **15** | (A1) 4,7-Dichlor-1-ethyl-chinolinium-tetrafluoroborat: 0,78 g (A2) 1,3-Diamino-4-(2-hydroxy-ethoxy)-benzol-sulfat: 0,67 g PHₘ = 10,0 | gelb | 80,78 | -3,37 | +60,74 |
| **16** | (A1) 4,7-Dichlor-1-ethyl-chinolinium-tetrafluoroborat: 0,78 g (A2) 1,4-Diamino-2-methyl-benzol-sulfat: 0,55 g pHₘ = 10,0 | gelb | 73,93 | +8,79 | +73,69 |
| **17** | (A1) 4-Chlor-1 -ethyl-chinolinium-tetrafluoroborat: 0,70 g (A2) 5-Amino-2-methyl-phenol: 0,31 g pHₘ=10,5 | gelb | 77,67 | +0,82 | +30,07 |
| **18** | (A1) 4-Chlor-1 -ethyl-chinolinium-tetrafluoroborat: 0,70 g (A2) 5-((2-hydroxyethyl)amino)-2-methoxy-anilin-sulfat(1:1 )-hydrat(1:1 ): 0,75 g pHₘ=11 | braun | 53,89 | +5,06 | +12,89 |
| **19** | (A1) 4-Chlor-1-ethyl-chinolinium-tetrafluoroborat: 0,70 g (A2) 2,6,8-Trihydroxy-purin: 0,42 g pHₘ=11 | rosa | 57,75 | +30,42 | -3,83 |
| **20** | (A1) 4,7-Dichlor-1-ethyl-chinoliniumtetrafluoroborat. 0.79 g (A2) 1,4-Diamino-2-methyl-benzol-sulfat: 0.55 g | gelb-orange | 73,94 | 8,79 | 73,69 |
| **21** | (A1) 4,7-Dichlor-1-ethyl-chinoliniumtetrafluoroborat. 0.79 g (A2) 2-(2,4-Diaminophenoxy)etha-nol: 0.40 g | hellgelb | 80,78 | -3,37 | 60,74 |
| **22** | (A1) 4-Chlor-1-ethyl-chinolinium-tetrafluoroborat: 0,70 g (A2) 1-Ethyl-4-methyl-Chinolinium-tetrafluoroborat: 0,65 g | königs-blau | 42,03 | +17,75 | -45,36 |
| **23** | (A1) 4,7-Dichlor-1-ethyl-chinoliniumtetrafluoroborat. 0.78 g (A2) 1-Ethyl-4-methyl-Chinolinium-tetrafluoroborat: 0,65g | königsblau | 41,96 | 18,11 | -46,30 |
| **24** | (A1 ) 4,7-Dichlor-1-ethyl-chinoliniumtetrafluoroborat. 0.78 g (A2) 1 -Ethyl-2-methyl-Chinolinium-jodid: 0,75 g | pink | 45,47 | 54,39 | -26,87 |

## Patentansprüche

1. Mittel zur Färbung von Fasern (A), welches durch Vermischen zweier Komponenten -gegebenenfalls unter Zusatz eines Alkalisierungsmittels oder einer Säure- erhalten wird, und **dadurch gekennzeichnet ist, dass** die eine Komponente (Komponente A2) mindestens eine Verbindung mit nukleophilem Reaktionszentrum enthält und die andere Komponente (Komponente A1) mindestens ein 1-Alkyl-chinolinium-Derivat der Formel (la) oder (Ib) enthält, 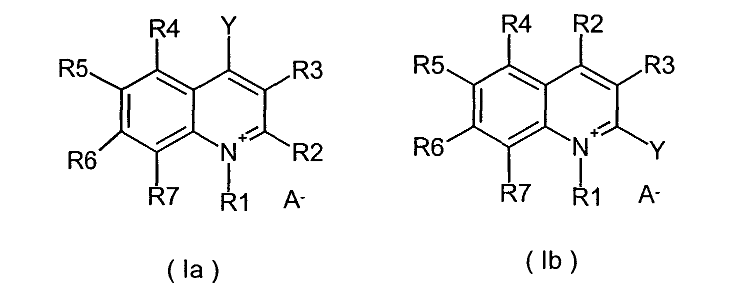 wobei gilt:
**R1** ist gleich einer geradkettigen oder verzweigten C1- bis C8-Alkylgruppe, einer C1- bis C8-Monohydroxyalkylgruppe, einer C2- bis C8-Polyhydroxyalkylgruppe oder einer C1- bis C8-Alkoxy-(C1- bis C8)-alkylgruppe;
**R2, R3, R4, R5, R6,** und **R7** können gleich oder verschieden sein und sind unabhängig voneinander gleich einem Wasserstoffatom, einer geradkettigen oder verzweigten C1- bis C4-Alkylgruppe, einer geradkettigen oder verzweigten C1- bis C4-Hydroxyalkylgruppe, einer Hydroxygruppe, einer Methoxygruppe, einer Ethoxygruppe, einer Benzylgruppe, einem Halogenatom, einer Nitrogruppe, einer Nitrosogruppe, einer Cyanogruppe, einer Trifluormethylgruppe, einer -COOH -Gruppe, einer -CO₂R^{a}-Gruppe, einer -CONHR^{a}-Gruppe, einer -CON(R^{a})₂-Gruppe, einer ―O(CO)R^{a}-Gruppe einer -O-SO₂CF₃-Gruppe, einer -OCH₂Aryl -Gruppe, einer -SO₂NH₂-Gruppe, einer -SO₂CHF₂-Gruppe, einer-SO₂CF₃-Gruppe, einer SO₂NH₂-Gruppe, einer SO₂NHR^{a}-Gruppe, einer SO₂N(R^{a})₂-Gruppe, einer SO₂R^{a}-Gruppe, einer -NH₂ -Gruppe, einer -NHR^{a} -Gruppe, einer -N(R^{a})₂-Gruppe, einer-NHCOR^{a}-Gruppe, einer-NHCOOR^{a}-Gruppe, einer -CH₂NH₂-Gruppe, einer-CH₂NHR^{a}-Gruppe, einer-CH₂N(R^{a})₂-Gruppe oder einer -PO(OR^{a})₂-Gruppe, wobei R^{a} gleich einem Wasserstoffatom, einem gegebenenfalls substituierten aromatischen Carbozyklus oder Heterozyklus oder einer C1- bis C6-Alkylgruppe ist;
**Y** steht für ein Jodatom, Bromatom, Chloratom oder eine Ethoxygruppe, Phenoxygruppe, CF₃-SO₂-O-Gruppe, Aryl-SO₂-O-Gruppe, (-SO₃)-Gruppe; und
**A**⁻ ist gleich dem Anion einer organischen oder anorganischen Säure.

2. Mittel nach Anspruch 1, **dadurch gekennzeichnet, dass** die Verbindung der Formel (I)/(Ia) ausgewählt ist aus 4-Chloro-1-ethylchinoliniumsalzen, 4,7-Dichloro-1-ethyl-chinoliniumsalzen, 4-Chloro-1 -ethyl-7-trifluoromethyl-chinoliniumsalzen, 4-Chloro-1-ethyl-6-nitro-chinoliniumsalzen, 4-Chloro-1-methyl-chinolinium-salzen, 4-Methoxy-1-methylchinoliniumsalzen, 4-Ethoxy-1 -methyl-chinoliniumsalzen, 4-Ethoxy-1 -ethylchinoliniumsalzen, 4-Jodo-1-methyl-chinoliniumsalzen, 4-Chloro-1-methyl-2-phenyl-chinoliniumsalzen, 4-Chloro-1-methyl-3-[(methylphenyl-amino)-sulfonyl]-chinoliniumsalzen, 4-Chloro-2-[[[4-(dimethylamino)phenyl]-imino]-methyl]-6-methoxy-1-methyl-chinoliniumsalzen, 4-Chloro-1-ethyl-3-[(phenylamino)sulfonyl]-chinoliniumsalzen, 4-Chloro-6-dimethyl-carbamoyl-1-methyl-chinoliniumsalzen, 4-Chloro-1-ethyl-6-sulfamoyl-chinoliniumsalzen, 4-Chloro-1-ethyl-7-nitro-chinolinium-salzen, 4-Chloro-1-ethyl-7-methoxychinoliniumsalzen, 2-Chloro-1-methyl-chinoliniumsalzen, 2,6-Dichloro-1-methyl-chinoliniumsalzen, 2-Chloro-1-methyl-4-trifluoro-methansulfonyloxychinoliniumsalzen und 1 -Ethyl-4-(toluol-4-sulfonyloxy)-chinoliniumsalzen.

3. Mittel nach Anspruch 2, **dadurch gekennzeichnet, dass** die Verbindung der Formel (I)/(Ia) ausgewählt ist aus 4-Ethoxy-1-ethylchinolinium-tetrafluoroborat, 4-Chloro-1-ethyl-chinolinium-tetrafluoroborat 4,7-Dichloro-1-ethyl-chinolinium-tetrafluoroborat, 4-Chloro-1-ethyl-7-trifluoromethyl-chinolinium-tetrafluoroborat, 4-Chloro-1-ethyl-6-nitrochinolinium-tetrafluoroborat, 4-Chloro-1-methyl-chinolinium-chlorid, 4-Jodo-1 -methyl-chinoliniumjodid, 4-Chloro-1-methyl-2-phenyl-chinoliniumtetrafluoroborat, 4-Chloro-1-methyl-3-[(methylphenyl-amino)sulfonyl]-chinolinium-methylsulfat, 4-Chloro-2-[[[4-(dimethyl-amino)phenyl]imino]-methyl]-6-methoxy-1-methyl-chinolinium-chlorid, 4-Chloro-1-ethyl-3-[(phenylamino)-sulfonyl]-chinolinium-tetrafluoroborat, 4-Chloro-6-dimethylcarbamoyl-1 -methyl-chinolinium-tetrafluoroborat, 4-Chloro-1 -ethyl-6-sulfamoyl-chinolinium-tetrafluoroborat, 4-Chloro-1-ethyl-6-formylchinolinium-tetrafluoroborat, 4-Chloro-1-ethyl-7-nitro-chinoliniumtetrafluoroborat, 4-Chloro-1-ethyl-7-methoxy-chinolinium-tetrafluoroborat, 2-Chloro-1 -methyl-chinolinium-tetrafluoroborat, 2,6-Dichloro-1-methylchinolinium-tetrafluoroborat, 1-Ethyl-4-(toluene-4-sulfonyloxy)-chinoliniumchlorid, 1-Ethyl-4-(toluene-4-sulfonyloxy)-chinolinium-tetrafluoroborat, 1-Ethyl-4-chinoliniumsulfonat und 2-Chloro-1-methyl-4-trifluoromethansulfonyloxy-chinolinium-tetrafluoroborat.

4. Mittel nach Anspruch 1, **dadurch gekennzeichnet, dass** die Verbindung mit nukleophilem Reaktionszentrum ausgewählt ist aus aliphatischen oder aromatischen Verbindungen mit einer oder mehreren Aminogruppen, aliphatischen oder aromatischen Verbindungen mit einer Hydrazingruppe, stickstoffhaltigen Heterozyklen, Aminosäuren, Oligopeptiden mit 2 bis 9 Aminosäuregruppen, aromatische Hydroxyverbindungen und CH-aktiven Verbindungen.

5. Mittel nach einem der Ansprüche 1 bis 4, **dadurch gekennzeichnet, dass** das gebrauchsfertige Färbemittel (A) einen pH-Wert von 3 bis 11 aufweist.

6. Mittel nach einem der Ansprüche 1 bis 5, **dadurch gekennzeichnet, dass** das 1-Alkyl-chinolinium-Derivat der Formel (I)/(Ia) in der Komponente (A1) in einer Gesamtmenge von 0,02 bis 20 Gewichtsprozent und die Verbindung mit nukleophilem Reaktionszentrum in der Komponente (A2) in einer Gesamtmenge von 0,02 bis 20 Gewichtsprozent enthalten ist.

7. Mittel nach einem der Ansprüche 1 bis 6, **dadurch gekennzeichnet, dass** es das 1-Alkyl-chinolinium-Derivat der Formel (I)/(Ia) und die Verbindung mit nukleophilem Reaktionszentrum, bezogen auf die gebrauchsfertige Zubereitung, jeweils in einer Gesamtmenge von 0,01 bis 10 Gewichtsprozent enthält.

8. Mittel nach einem der Ansprüche 1 bis 7, **dadurch gekennzeichnet, dass** das gebrauchsfertige Färbemittel (A) sowie die Komponenten (A1) und (A2) jeweils in Form einer Lösung, einer Emulsion, eines Schaumes, einer Creme oder eines Gels vorliegen.

9. Mittel nach einem der Ansprüche 1 bis 8, **dadurch gekennzeichnet, dass** die Komponente (A1) und/oder (A2) zusätzlich mindestens einen direktziehenden Farbstoff aus der Gruppe der sauren oder basischen Farbstoffe, Nitrofarbstoffe, Azofarbstoffe, Chinonfarbstoffe und Triphenylmethanfarbstoffe enthält.

10. Mittel nach einem der Ansprüche 1 bis 9, **dadurch gekennzeichnet, dass** die Komponente (A1) und/oder (A2) aus mehreren Einzelkomponenten besteht.

11. Mittel nach einem der Ansprüche 1 bis 10, **dadurch gekennzeichnet, dass** es ein Haarfärbemittel ist.

12. Mehrkomponenten-Kit, bestehend aus einer ein 1-Alkyl-chinolinium-Derivat der Formel (I)/(Ia) enthaltenden Komponente (A1) und einer eine Verbindung mit nukleophilem Reaktionszentrum enthaltenden Komponente (A2).

13. Mehrkomponenten-Kit bestehend aus einer ein 1-Alkyl-chinolinium-Derivat der Formel (I)/(Ia) enthaltenden Komponente (A1) und einer eine Verbindung mit nukleophilem Reaktionszentrum enthaltenden Komponente (A2) sowie einer ein Alkalisierungsmittel oder eine Säure enthaltenden Komponente (A3).

14. Verfahren zum Färben von Fasern, **dadurch gekennzeichnet, dass** man ein Mittel nach einem der Ansprüche 1 bis 11 auf die Faser aufträgt, für eine Dauer von 5 bis 60 Minuten bei einer Temperatur von 20 bis 50 °C auf die Faser einwirken läßt, sodann die Faser mit Wasser spült und anschließend trocknet.
